(19) Europäisches Patentamt
European Patent Office
Office européen des brevets

(11) Publication number: **0 200 960**
**A1**

(12) **EUROPEAN PATENT APPLICATION**

(21) Application number: **86105153.0**

(22) Date of filing: **15.04.86**

(51) Int. Cl.[4]: **G 01 N 33/74**, G 01 N 33/533, C 07 J 41/00, C 07 J 1/00, C 07 J 17/00

(30) Priority: **08.05.85 US 732463**

(43) Date of publication of application: **12.11.86**
**Bulletin 86/46**

(84) Designated Contracting States: **BE DE FR IT**

(71) Applicant: **ABBOTT LABORATORIES, 14th Street and Sheridan Road North St, North Chicago Illinois 60064 (US)**

(72) Inventor: **Vanderbilt, Anne Symon, 1509 McAree Road, Waukegan Illinois 60085 (US)**
Inventor: **Osikowicz, Eugene Walter, 1549 Eagle Street, Melrose Park Illinois 60160 (US)**
Inventor: **Fino, James Robert, 231 Southwick Court, Vernon Hills Illinois 60061 (US)**
Inventor: **Shipchandler, Mohammed Tyebji, 640 Burdick Street, Libertyville Illinois 60048 (US)**

(74) Representative: **Modiano, Guido et al, MODIANO, JOSIF, PISANTY & STAUB Modiano & Associati Via Meravigli, 16, I-20123 Milan (IT)**

(54) **Total estriol fluorescence polarization immunoassay.**

(57) The present invention is directed to a homogeneous fluorescence polarization immunoassay for determining total estriol content in body fluids, to the various components needed for preparing and carrying out such an assay, and to methods of making these components. Specifically, tracers, immunogens and antibodies are disclosed, as well as methods for preparing them. The assay is conducted by measuring the degree of polarization of plane polarized light that has been passed through a sample containing antiserum and tracer. The combination of antiserum and tracer used will possess excellent cross-reactivity to estriol-3-sulfate, eliminating the need for enzyme hydrolysis of this conjugate to free estriol.

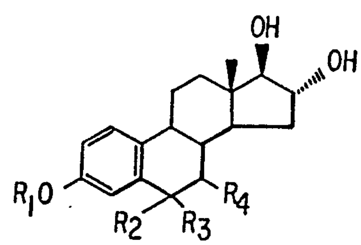

EP 0 200 960 A1

# TOTAL ESTRIOL FLUORESCENCE POLARIZATION IMMUNOASSAY

## BACKGROUND OF THE INVENTION

### Technical Field

The present invention relates to a method and reagents for a fluorescence polarization immunoassay procedure for determining the amount of estriol and estriol conjugates in pregnancy serum, plasma, or urine. More specifically, the invention relates to reagents (tracers and antibodies) for determining the amount of estriol and estriol conjugates in a sample, immunogen compounds used to raise antibodies, and analytical methods for conducting the assay.

### Background Art

The production of estriol by the fetus and placenta forms the basis of one of the most commonly used fetoplacental monitoring tests. Consistently low estriol levels throughout pregnancy, or a sudden drop observed in serial determinations, is associated with fetal distress. Levels of estriol in serum and urine normally increase rapidly during the third trimester. In pregnancies complicated by conditions such as toxemia, hypertension, diabetes and post maturity syndrome, serial estriol determinations may be useful to assess fetal well-being.

One of the many functions of the liver is the conjugation of various drugs and physiological substances into sulfate and glucuronide derivatives. Estriol (abbreviated as E3) from the fetoplacental unit is conjugated in the maternal liver at the C-3 position

with either sulfate (E3-3S) or glucuronide (E3-3G) and is conjugated with glucuronide at the C-16 position (E3-16G); a double conjugate having sulfate at the C-3 position and glucuronide at the C-16 position (E3-3S-16G) is also formed. Measurement of total estriol in a biological fluid requires quantification of both "free", or unconjugated, estriol and all estriol conjugates in that sample. Measurement of "total urinary estriol", for example, predominantly reflects excretion of monoconjugated C-3 and C-16 glucuronides, which are not bound to albumin as are the sulfate conjugates that remain behind in plasma and constitute a major portion of "total serum estriol".

In the past, total estriol levels have typically been measured by high performance liquid chromatography (HPLC), gas chromatography (GC), enzyme immunoassay (EIA) and radioimmunoassay (RIA). Generally, these methods employ an enzyme mixture containing both a ß-glucuronidase and a sulfatase to hydrolyze the estriol conjugates prior to quantitation of total estriol. While reasonably specific for detecting estriol levels, these methods are not without drawbacks. High performance liquid chromatography and gas chromatography require sample extraction procedures and the assay time is lengthy. Enzyme immunoassays are characterized by several disadvantages: 1) the reagents are relatively unstable; 2) any components in the biological samples which may influence the enzyme reaction in the assay (such as enzyme inhibitors or enzymes which catalyze similar reactions) will affect the assay results; and 3) any compounds in the biological samples which may affect the absorbance measured in an enzyme immunoassay (e.g., hemoglobin, bilirubin or other chromophores) will affect the accuracy of the results. Radioimmunoassays are characterized by several disadvantages as well,

including relatively short reagent shelf life due to isotopic decay and problems related to disposal of radioactive materials following use in the assay.

In general, competitive binding immunoassays have provided a preferable alternative to chemical methods such as gas chromatography and high pressure liquid chromatography. Typically, competitive binding immunoassays are used for measuring ligands in a test sample. (For purposes of this disclosure, a "ligand" is a substance of biological interest to be determined quantitatively by a competitive binding immunoassay technique.) The ligands compete with a labeled reagent, or "ligand analog," or "tracer," for a limited number of receptor binding sites on antibodies specific to the ligand and ligand analog. The concentration of ligand in the sample determines the amount of ligand analog which binds to the antibody: the amount of ligand analog that will bind is inversely proportional to the concentration of ligand in the sample, because the ligand and the ligand analog each bind to the antibody in proportion to their respective concentrations.

Fluorescence polarization provides a quantitative means for measuring the amount of tracer-antibody conjugate produced in a competitive binding immunoassay. Fluorescence polarization techniques are based on the principle that a fluorescent labeled compound, when excited by plane polarized light, will emit fluorescence having a degree of polarization inversely related to its rate of rotation. Accordingly, when a tracer-antibody conjugate having a fluorescent label is excited with plane polarized light, the emitted light remains highly polarized because the fluorophore is constrained from rotating between the time that light is absorbed and emitted. In contrast, when an unbound tracer is excited by plane polarized light, its rotation is much faster than that of the corresponding

tracer-antibody conjugate. As a result, the light emitted from the unbound tracer molecules is depolarized.

Fluorescence polarization immunoassay techniques are well known in the art; however, to date the use of such techniques for the determination of total estriol levels has not been described.

The present invention offers an advance in the art in that highly accurate determinations of total estriol concentration in serum, plasma, or urine are obtainable with increased convenience and without the disadvantages heretofore imposed by the various available assay systems. The assay conducted in accordance with the present invention is particularly accurate, as will be explained infra.

SUMMARY OF THE INVENTION

The present invention is directed to a fluorescence polarization assay for total estriol; to tracers, immunogens and antibodies for use in the assay; and to analytical methods for conducting the assay.

A first aspect of the invention relates to the discovery of certain tracers and immunogens having novel structures. According to this first aspect of the invention, both the tracers and immunogens can be represented by the structural formula shown in Figure 7 of the drawings, wherein $R_1$ is H or R-Z-Q; $R_2$ and $R_3$ are H when $R_1$ is R-Z-Q and are taken together as R-Z-Q when $R_1$ and $R_4$ are H; $R_4$ is R-Z-Q when $R_1$, $R_2$ and $R_3$ are H and is H when either $R_1$ or $R_2$ taken together with $R_3$ is R-Z-Q;

Q is a poly (amino acid), a poly (amino acid) derivative or another immunologically active carrier, or fluorescein or a fluorescein derivative;

Z is CO, NH, $CH_2NH$ or CS when Q is fluorescein or a fluorescein derivative and is N, NH, $SO_2$, $PO_2$, PSO or a glucuronide moiety when Q is a

poly (amino acid), a poly (amino acid) derivative or another immunologically active carrier; and

R is a linking group including up to 7 heteroatoms when Q is a poly (amino acid), a poly (amino acid) derivative or other immunologically active carrier and including up to 10 heteroatoms when Q is fluorescein or a fluorescein derivative, and having a total of from 0 to 20 carbon atoms and heteroatoms arranged in a straight or branched chain and containing up to two ring structures.

A second aspect of the invention relates to antibodies raised by the novel immunogen. According to the second aspect of the invention, antibodies are prepared in response to a compound according to Claim 1 when Q is a poly (amino acid) or a derivative thereof or other immunologically active carrier.

According to a third aspect of the invention, an immunogen is made by a method comprising the step of coupling a compound represented by the structural formula shown in Figure 7 wherein $R_1$ is H or R-X; $R_2$ and $R_3$ are H when $R_1$ is R-X and are taken together as R-X when $R_1$ and $R_4$ are H; $R_4$ is R-X when $R_1$, $R_2$ and $R_3$ are H and is H when either $R_1$ or $R_2$ taken together with $R_3$ is R-X;

X is $NH_2$, $SO_3H$, $PO_3H$, $PSO_2H$ or a glucuronide moiety when $R_2$, $R_3$ and $R_4$ are H, is $NH_2$ or COOH when $R_1$, $R_2$ and $R_3$ are H and is $NH_2$ when $R_1$ and $R_4$ are H; and

R is a linking group including up to 7 heteroatoms and having a total of from 0 to 20 carbon atoms and heteroatoms arranged in a straight or branched chain and containing up to two ring structures

with a poly (amino acid), a poly (amino acid) derivative or another immunologically active carrier.

According to a fourth aspect of the invention, a method is provided for making a tracer by coupling a

compound represented by the structural formula shown in Figure 7, wherein: $R_1$ is H or R-Y; $R_2$ and $R_3$ are H when $R_1$ is R-Y and are taken together as R-Y when $R_1$ and $R_4$ are H; $R_4$ is R-Y when $R_1$, $R_2$ and $R_3$ are H and is H when either $R_1$ or $R_2$ taken together with $R_3$ is R-Y.

Y is $NH_2$, COOH, $SO_3H$, $SO_2Cl$, SH, CHO, CN, OH or a glucuronide moiety; and

R is a linking group including up to 10 heteroatoms and having a total of from 0 to 20 carbon atoms and heteroatoms arranged in a straight or branched chain and containing up to two ring structures

with fluorescein or a derivative of fluorescein.

A fifth aspect of the invention relates to a reagent system for use in conducting a fluorescence polarization immunoassay comprising the combination of a novel fluorescein tracer compound with an antibody having a cross-reactivity with estriol, estriol-3-sulfate and the novel tracer compound of 100% ± 10%. This cross-reactivity of 90-110% eliminates the need to enzymatically hydrolyze the sulfate conjugate to free estriol and thus makes possible for the first time a homogeneous fluorescence polarization immunoassay for total estriol, as will be explained _infra_.

A sixth aspect of the invention relates to an improved process for measuring the concentration of total estriol. A sample is contacted with estriol antiserum, ß-glucuronidase, and a fluorescein-containing estriol derivative capable of producing a detectable fluorescence polarization response to the presence of the estriol antiserum. Plane polarized light is then passed through the solution to obtain a fluorescence polarization response, and this response is detected as a measure of the amount of total estriol in the sample.

Further objects and attendant advantages of the invention will be best understood from a reading of the following detailed description taken together with the figures and the examples.

BRIEF DESCRIPTION OF THE DRAWINGS

Figure 1 shows the general structure of the estriol to be quantitatively determined in accordance with the present invention.

Figure 2 shows a general structural formula for the conjugated forms of estriol, wherein estriol is derivatized at the C-3 and C-16 positions.

Figure 3 shows the structure of estriol-3-sulfate (E3-3S).

Figure 4 shows the structure of estriol-3-glucuronide (E3-3G).

Figure 5 shows the structure of estriol-16-glucuronide (E3-16G).

Figure 6 shows the structure of estriol-3-sulfate-16-glucuronide (E3-3S-16G).

Figure 7 shows a general structural formula for the tracers and the immunogens of the present invention as well as the classes of reactants used in preparing them.

Figure 8 shows the alternate structural formulae and names of the fluorescein moiety included in the tracers of the present invention.

Figures 9 through 31 show various examples of structures of tracers in accordance with the present invention.

Figure 32 shows an example of an immunogen in accordance with the present invention.

DETAILED DESCRIPTION OF THE INVENTION

The present invention involves the use of fluorescein and derivatives of fluorescein. In particular, a necessary property of fluorescein and its derivatives for the usefulness of the tracer compounds is the fluorescence of fluorescein. Fluorescein exists in either of two tautomeric forms, depending on the acid concentration (pH) of the environment. In the open (acid) form, there are a number of conjugated double bonds which make that form of fluorescein (and compounds containing a fluorescein moiety) capable of absorbing blue light and emitting green fluorescence after an excited state lifetime of about four nanoseconds. When the open and closed forms coexist, the relative concentration of molecules in the open and closed forms is easily altered by adjustment of the pH level. Generally, the tracer compounds of the present invention exist in solution as biologically acceptable salts such as sodium, potassium, ammonium and the like, allowing the compounds to exist in the open, fluorescent form, when employed in the analytical methods of the present invention. The specific salt present will depend on the buffer employed to adjust the pH level. For example, in the presence of a sodium phosphate buffer, the compounds of the present invention will generally exist in the open form, as a sodium salt.

As used herein, the term "fluorescein," either as an individual compound or as a component of a larger compound, is meant to include both the open and closed forms, if they exist for a particular molecule, except in the context of fluorescence. An open form is necessary for the fluorescence to occur.

The numbering of carbon atoms of the fluorescein molecule varies, depending upon whether the

open or closed form of the molecule is considered. Accordingly, the literature concerning fluorescein and its compounds is not uniform as to carbon atom numbering. The numbering system used herein is illustrated in Figure 8 as the lactone form of fluorescein. The tracers of the present invention will be referred to as 3-position, 6-position or 7-position tracers in accordance with this numbering system.

A tracer in solution which is not complexed to an antibody is free to rotate in less than the time required for absorption and re-emission of fluorescent light. As a result, the re-emitted light is relatively randomly oriented so that the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the resulting mixture of the free tracer and tracer-antibody complex assumes a value intermediate between that of the tracer and that of the tracer-antibody complex. If a sample contains a high concentration of the ligand, the observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertically polarized component of the emitted light, the polarization of fluorescence in the reaction mixture may be accurately determined.

The Reagents

Both the immunogens and the tracers of the present invention can be represented by the general structural formula shown in Figure 7 of the drawings, wherein $R_1$ is H or R-Z-Q; $R_2$ and $R_3$ are H when $R_1$ is R-Z-Q and are taken together as R-Z-Q when $R_1$ and $R_4$ are H; $R_4$ is R-Z-Q when $R_1$, $R_2$ and $R_3$ are H and is H when either $R_1$ or $R_2$ taken together with $R_3$ is R-Z-Q;

Q is a poly (amino acid), a poly (amino acid) derivative or another immunologically active carrier, or fluorescein or a fluorescein derivative;

Z is CO, NH, $CH_2NH$ or CS when Q is fluorescein or a fluorescein derivative and is N, NH, $SO_2$, $PO_2$, PSO or a glucuronide moiety when Q is a poly (amino acid), a poly (amino acid) derivative or another immunologically active carrier; and

R is a linking group including up to 7 heteroatoms when Q is a poly (amino acid), a poly (amino acid) derivative or other immunologically active carrier and including up to 10 heteroatoms when Q is fluorescein or a fluorescein derivative, and having a total of from 0 to 20 carbon atoms and heteroatoms arranged in a straight or branched chain and containing up to two ring structures.

The objective is to have competition between estriol (including estriol-3-sulfate) and the tracer for the recognition sites of the antibody. Great variations in the structure of the haptens and tracers are allowed in achieving this goal. For the purposes of this invention, "haptens" are precursors of the immunogens comprising generally a substituted estriol derivative bearing a group suitable for linking to an immunologically active carrier.

## The Structure of the Immunogens

Usable antibodies can be produced from a variety of estriol derivatives. Immunogens prepared from estriol compounds functionalized at the C-3, -6 or -7 position can produce antibodies in animals. Such antibodies are useful in a total estriol assay according to the invention when combined with an appropriate tracer.

The immunogens of the present invention are derived from the general structure shown in Figure 4 and can be prepared by coupling a compound of the class shown with a poly (amino acid) or a derivative of a poly (amino acid) or other immunologically active carrier as will be discussed in the context of the synthetic method.

In a preferred form of the invention, the immunogen is prepared by coupling the aforedescribed substituted estriol compound with bovine serum albumin. Various other protein carriers may also be used to good advantage, e.g., keyhole limpet hemocyanin, egg ovalbumin, bovine gamma-globulin, thyroxine-binding globulin, and so forth. Alternatively, synthetic poly (amino acids) having a sufficient number of available amino groups can be employed, as can other synthetic or natural polymeric materials bearing functional groups reactive with estriol.

## The Structure of the Tracers

The tracers of the present invention have the general formula shown in Figure 7. The tracer is an estriol derivative that is linked to a fluorescein derivative by, for example, an amido aminoalkyl, thiourea, ether, thioether, or oximo group, as will be discussed in the context of the synthetic method.

By way of example, any of the following fluorescein derivatives can be used:

| | |
|---|---|
| $Fl-NH_2$ | fluorescein amine |
| $Fl-CO_2H$ | carboxyfluorescein |
| $Fl-NHCOCH_2I$ | -iodacetamidofluorescein |
| $Fl-NHCOCH_2Br$ | -bromoacetamidofluorescein |
| 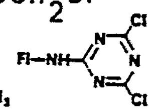 | (DTAF) 2, 4-dichloro-1,3,5,-triazin-2-ylamino-fluorescein |
| 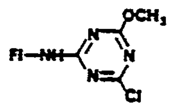 | 4-chloro-6-methoxy-1,3,5-triazin-2-ylamino-fluorescein |
| Fl-NCS | fluorescein thioisocyanate |
| $Fl-CH_2NH_2$ | 4'-aminomethylfluorescein |

The Antibodies

The antibodies of the present invention are prepared by eliciting a response in rabbits to the immunogens described supra. The immunogen is administered to animals or to in vitro cultures of immunocompetent cells by a series of inoculations, in a manner well known to those skilled in the art. It should be understood that although rabbits were the preferred immune host to estriol immunogens in the experiments detailed herein, any in vivo or in vitro host capable of producing antibodies to the structures herein outlined may be employed.

Synthesis of the Immunogens

The immunogens of the present invention are prepared by coupling a hapten having the general structure of Figure 7 wherein $R_1$ is H or R-X; $R_2$ and $R_3$ are H when $R_1$ is R-X and are taken together as R-X when $R_1$ and $R_4$ are H; $R_4$ is R-X when $R_1$,

$R_2$ and $R_3$ are H and is H when either $R_1$ or $R_2$ taken together with $R_3$ is R-X;

X is $NH_2$, $SO_3$, $PO_4$, $PSO_3$ or a glucuronide moiety; and

R is a linking group including up to 7 heteroatoms and having a total of from 0 to 20 carbon atoms and heteroatoms arranged in a straight or branched chain and containing up to two ring structures

to a poly (amino acid), poly (amino acid) derivative or other immunologically active "carrier." The hapten can be linked to the carrier by an amide or amide-like linkage.

In a preferred embodiment, the carrier is bovine serum albumin and the hapten has the structure of Figure 7 where $R_1$ is H, $R_2$ is $N-O-CH_2COOH$, and $R_3$ is H.

The hapten is coupled to the poly (amino acid) or other carrier through its acid group. This group can be activated either prior to its addition to the carrier or in situ with the carrier, utilizing well known carbodiimide procedures and reagents such as N,N'-dicyclohexylcarbodiimide or 1-ethyl-3-(3-dimethylaminopropyl) carbodiimide. In a preferred embodiment, the hapten is activated prior to its addition to the carrier by means of N,N'-dicyclohexylcarbodiimide and N-hydroxysuccinimide in an organic medium. The activated hapten is added to a carrier which has been dissolved in suitable buffer, e.g., phosphate or borate, at suitable pH, usually between 6 and 9.

The 3-position haptens utilized in immunogen preparation are synthesized by refluxing estriol together with a haloalkanoic ester and a suitable base. The ester is hydrolyzed by base, revealing the free carboxylic acid function. Other 3-position haptens,

such as the derivatives of sulfonates, phosphonates and glucuronides, could be utilized in the preparation of immunogens according to the present invention, as will be readily apparent to those skilled in the art.

The 6-position haptens utilized in immunogen preparation were made using a modification of the method of E.P. Burrows, D.L. di Pietro and H.E. Smith, Journal of Organic Chemistry, 37, 4000 (1972) to convert estriol to its 6-keto triacetate derivative. The method of E. Kuss and R. Goebel, Steroids, 511, (1972) converted the 6-ketoestriol triacetate to estriol-6-carboxymethyloxime.

The 7-position haptens utilized in immunogen preparation were prepared by reduction of the aforementioned 6-ketoestriol triacetate, using sodium borohydride, to the corresponding 6-hydroxy compound. Dehydration and reacetylation yielded 6-dehydroestriol triacetate. Addition of methyl-3-mercaptopropionate to the double bond, according to the method of C.E. Cook, G.R. Tallent, and H.P. Christensen, Life Sciences, 14,1075 (1974), gave 7-(2-methoxycarbonylethylmercapto) estriol triacetate. Hydrolysis of this compound yielded 7-(2-carboxyethylmercapto)estriol.

Synthesis of the Tracers

The tracers of the present invention are prepared by coupling a fluorescein moiety, or a derivative of fluorescein, to the general structure shown in Figure 7, wherein $R_1$ is H or R-Y; $R_2$ and $R_3$ are H when $R_1$ is R-Y and are taken together as R-Y when $R_1$ and $R_4$ are H; $R_4$ is R-Y when $R_1$, $R_2$ and $R_3$ are H and is H when either $R_1$ or $R_2$ taken together with $R_3$ is R-Y;

Y is $NH_2$, COOH, $SO_3H$, $SO_2Cl$, SH, CHO, CN, OH or a glucuronide moiety; and

R is a linking group including up to 10 heteroatoms and having a total of from 0 to 20 carbon atoms and heteroatoms arranged in a straight or branched chain and containing up to two ring structures.

In the presently preferred embodiment, the fluorescein derivative is 5-carboxyfluorescein, prepared as in Example 1. This is coupled to a precursor of the general structure of Figure 7, wherein $R_1$ is $CH_2CO-NH-CH_2CH_2NH_2$ and $R_2$, $R_3$ and $R_4$ are H, by first forming the active ester of 5-carboxyfluorescein. The preferred active ester is the N-hydroxysuccinimide active ester, and the preferred coupling method is via N,N'-dicyclohexylcarbodiimide activation in dry N,N-dimethylformamide. Other activating groups (such as N-hydroxybenzotriazole and pentachlorophenol) and solvents (such as pyridine, acetonitrile, and dioxane) can be used. The reactants are preferably coupled under conditions for forming amide linkages, and it is most preferred that active ester procedures be used. Usable tracers can be prepared from a variety of estriol derivatives.

Estriol derivatives with terminal amino groups are synthesized by coupling the terminal carboxylic acid groups with diamines, such as ethylenediamine and piperazine, using the aforementioned carbodiimide procedures. Active ester is prepared from the carboxylic acid group and then reacted with the diamine using well known methods for preparing amide bonds. The amine or imine is then coupled to fluorescein derivatives having functionalities reactive toward amines or imines, e.g., carboxyfluorescein active esters, fluorescein isothiocyanates, 2,4-dichloro-1,3,5-triazine-2-ylaminofluorescein, or alkoxy-2,4-dichloro-1,3,5-triazine-2-ylaminofluorescein. Estriol derivatives with an acid group, e.g., carboxylic

acid, sulfonate, phosphonate or glucuronide, are coupled to fluorescein derivatives having functionalities reactive toward these acids, e.g., aminofluorescein, 4'-aminomethylfluorescein, or a novel class of N-alkylated 4'-aminomethylfluorescein compounds (claimed in U.S. Patent Application ________________ filed concurrently herewith). The method of coupling is preferably the aforementioned carbodiimide procedure.

## The Assay

The particular tracers, antibodies and ß-glucuronidase enzyme of the present invention have been found to accurately and specifically measure levels of total estriol levels in serum, plasma and urine. Figure 1 shows the structure of estriol. Figure 2 indicates the positions at which estriol is conjugated. Figure 3 shows the structure of estriol-3-sulfate. Figures 4, 5 and 6 show the other forms of conjugated estriol which commonly occur, described supra. The assay of the present invention provides a more rapid and convenient total estriol assay method than prior art methods, in that the system requires no specimen treatment before analysis, utilizes reagents that are chemically and thermally stable, and eliminates the need for enzyme hydrolysis of estriol-3-sulfate conjugates.

In accordance with the analytical methods of the present invention, i.e., the methods of determining total estriol by a fluorescence immunoassay procedure using the tracer compounds and immunogens of the invention, a sample containing or suspected of containing estriol is intermixed with a biologically acceptable salt of a tracer, an antibody specific to estriol, estriol-3-sulfate and the tracer, and ß-glucuronidase. The antibody is produced using the immunogen as described above. The ß-glucuronidase

enzyme converts estriol glucuronide to free estriol. The free estriol and estriol-3-sulfate then compete with the tracer for limited antibody sites, resulting in the formation of complexes. By maintaining constant the concentration of tracer and antibody, the ratio of estriol and estriol-3-sulfate-antibody complex to tracer-antibody complex that is formed is directly proportional to the total amount of estriol in the sample. Therefore, upon exciting the mixture with linearly polarized light and measuring the polarization of the fluorescence emitted by a tracer and a tracer-antibody complex, one is able to determine quantitatively the total amount of estriol in the sample.

The results can be quantified in terms of net millipolarization units, span (in millipolarization units) and relative intensity. The measurement of millipolarization units indicates the maximum polarization when a maximum amount of the tracer is bound to the antibody in the absence of any estriol or estriol-3-sulfate. The higher the net millipolarization units, the better the binding of the tracer to the antibody. The span is an indication of the difference between the net millipolarizations at the points of maximum and minimum amount of tracer bound to the antibody. A larger span provides for a better numerical analysis of data. The intensity is a measure of the strength of the signal above background. Thus, a higher intensity will give a more accurate measurement. The intensity is determined at about 0.5 to 2.0 nanomolar for the preferred tracers of the invention, as the sum of the vertically polarized intensity plus twice the horizontally polarized intensity. The intensity of the tracer signal can range from about three times to about thirty times the background noise, depending upon the concentration of the tracer and other assay variables.

For the purposes of the present invention, an intensity of at least eight to ten times that of background noise is preferred.

Tables I, II and III show the results obtained with various embodiments of the present invention in terms of span and estriol-3-sulfate cross-reactivity. Table I shows data for assays produced by pairing antiserum raised against estriol-6-carboxymethyloxime-bovine serum albumin with two of the novel tracers of this invention, one 3-position tracer and one 6-position tracer. Table II shows that several of the 3-position tracers of this invention can be employed with appropriate antisera raised against immunogens of the prior art. Table III shows results for antisera from three different types of animals (rabbits, goats and sheep) prepared from a 7-position immunogen and paired with a 7-position tracer.

TABLE I

TOTAL ESTRIOL ASSAY: COMPARISON OF TWO TRACERS USING ANTISERUM AGAINST ESTRIOL-6-CARBOXYMETHYLOXIME-BOVINE SERUM ALBUMIN

| Rabbit Antiserum | Estriol-6-carboxymethyloxime-4'-N-ethylaminomethylfluorescein | | Estriol-3-carboxymethylether-ethylenediamine-5-carboxyfluorescein | |
|---|---|---|---|---|
| 1 | 100 | 10 | 150 | 150 |
| 2 | 100 | 40 | 130 | 95 |
| 3 | 104 | 12 | 60 | -- |
| 4 | 64 | 07 | 107 | -- |
| 5 | 96 | 13 | 110 | 150 |
| 6 | 90 | 50 | 97 | -- |
| 7 | 60 | 20 | 77 | -- |
| 8 | 62 | 15 | 103 | 150 |
| 9 | 33 | 34 | 87 | -- |
| 10 | 90 | 40 | 108 | 100 |
| 11 | 58 | 20 | 60 | 60 |
| 12 | 58 | 100 | 90 | -- |
| 13 | 77 | 87 | 130 | 130 |
| 14 | 65 | 12 | 75 | -- |
| 15 | 44 | 60 | 62 | -- |
| 16 | 100 | 110 | 130 | 100 |

Signal:noise $\leq$ 10

As these data illustrate, excellent signal-to-noise ratio, span, and cross-reactivity to estriol-3-sulfate can be obtained with several different tracer/antiserum combinations. For example, antiserum 12 used in combination with estriol-6-carboxymethyloxime-4'-N-ethylaminomethylfluorescein results in a signal-to-noise ratio 10, span of 58, and cross-reactivity of 100%. Similarly, antisera 10 and 16, when used in conjunction with estriol-3-carboxymethylether-ethylenediamine-5-carboxyfluorescein, resulted in a signal-to-noise ratio 10, span greater than 100, and cross-reactivity of 100%.

TABLE II

| Antiserum | Span | X-Reactivity, E3-3S (%) |
|---|---|---|
| Rabbit A | 50 | 115 |
| B | 40 | 112 |
| Goat A | 84 | 10 |
| B | 43 | 90 |
| C | 103 | 57 |
| Sheep A | 65 | 110 |

Signal:noise $\leq$ 10

Table II again illustrates that certain antisera produced against the immunogens of the present invention were found to possess estriol-3-sulfate cross-reactivity of 100%. These particular results were achieved by utilizing antisera produced against 7-position immunogens in combination with 7-position tracers. In addition, various antisera produced according to the present invention could be combined to yield cross-reactivity more nearly approximating 100%. For instance, appropriate amounts of goat antiserum B and sheep antiserum A could be combined to form an antiserum with the desired cross-reactivity.

TABLE III

TOTAL ESTRIOL ASSAY: TRACER/ANTISERUM SELECTION

| Tracer | Antiserum 16 | | Antiserum 2 | | Antiserum 1 | |
|---|---|---|---|---|---|---|
| | Span (mp) | X-Reactivity, E3-3S (%) | Span (mp) | X-Reactivity E3-3S (%) | Span (mp) | X-Reactivity E3-3S (%) |
| A | 150 | 140 | -- | -- | 130 | 150 |
| B | 130 | 100 | 130 | 100 | 150 | 150 |
| C | 140 | 140 | -- | -- | 150 | 150 |
| D | 100 | 70 | -- | -- | 100 | 80 |

A = Estriol-3-carboxymethylether-4'-N-ethylaminomethylfluorescein

B = Estriol-3-carboxymethylether-ethylenediamine-5-carboxyfluorescein

C = Estriol-3-carboxymethylether-ethylenediamine-6-carboxyfluorescein

D = Estriol-3-carboxymethylether-piperazine-5-carboxyfluorescein

Signal:noise $\lesssim$ 10

These data illustrate the assay performance of various 3-position tracers with several 6-position antisera. For a given antiserum, changing the tracer may greatly alter the cross-reactivity. By trial and error in selecting among various tracers and antisera, appropriate combinations facilitating 100% estriol-3-sulfate cross-reactivity can be found. The preferred embodiment of Table III is the combination of estriol-3-carboxymethylether-ethylenediamine-5-carboxyfluorescein with antiserum 16, which was raised against estriol-6-carboxymethyloximebovine serum albumin immunogen.

Thus, an important aspect of the invention lies in the discovery that certain preferred combinations of specific estriol tracers and specific anti-estriol antisera possess approximately 100% cross-reactivity to estriol-3-sulfate. Tracer and antiserum combinations having either much less than or much greater than 100% cross-reactivity to estriol-3-sulfate are not useful in a total estriol fluorescence polarization immunoassay.

How closely the estriol-3-sulfate cross-reactivity needs to approach 100% will depend upon the fraction of estriol-3-sulfate derivatives in the particular sample to be tested as well as the accuracy required in a given circumstance. For instance, in urine samples, estriol-3-sulfate derivatives constitute only about 14% of the total estriol conjugates. If the estriol-3-sulfate cross-reactivity of a tracer/antiserum combination is only 50%, the error in measurement or recovery will be 50% x .14, or 7%. In contrast, for serum samples, in which estriol-3-sulfate derivatives constitute 55% of the total conjugates, a 27.5% error will result from the use of the same tracer/antiserum combination. Those skilled in the art will be able to determine the cross-reactivity required for a specific

application. In most instances, an antiserum/tracer pair yielding a cross-reactivity of 100% ± 10% will be preferred.

In addition to possessing excellent cross-reactivity to estriol-3-sulfate, the estriol tracer and anti-estriol antiserum combinations of the present invention must be characterized by minimal cross-reactivity to other steroids. Table IV illustrates the specificity results obtained with the preferred embodiment of the invention. Cross-reactivity was tested with other steroids whose chemical structure could potentially cause interference with the total estriol assay. Cross-reactivity of a compound was determined by assaying estriol-free pooled human serum spiked to various concentrations of the steroid to be tested. Percent cross-reactivity = 100 x (measured concentration of total estriol divided by the concentration of the test material).

TABLE IV

Total Estriol
Cross Reactivity Evaluation

| | Steroid | Level Tested (ng/ml) | % Cross Reactivity |
|---|---|---|---|
| 1. | Estrone ($E_1$) | 5,000 | 0.10 |
| 2. | Estradiol ($E_2$) | 5,000 | 0.40 |
| 3. | Progesterone | 10,000 | 0.05 |
| 4. | Testosterone | 10,000 | 0.05 |
| 5. | $E_1$-S | 500 | 1.00 |
| 6. | $E_2$-S | 500 | 1.00 |
| 7. | Dehydroepiandrosterone sulfate | 5,000 | 0.10 |
| 8. | Cortisol | 5,000 | 0.10 |
| 9. | 16,17 Epiestriol | 5,000 | 0.10 |
| 10. | 17 Epiestriol | 5,000 | 6.00 |

The preferred method of the improved assay of the present invention will now be discussed in detail. The assay is a "homogeneous assay," which means that the end polarization readings are taken from a solution in which bound tracer is not separated from unbound tracer. This is a distinct advantage over heterogeneous immunoassay procedures wherein the bound tracer must be separated from the unbound tracer before a reading can be taken.

The pH at which the method of the present invention is practiced must be sufficient to allow the fluorescein moiety of the tracers to exist in their open form. The pH may range from about 5 to 10, more preferably in the range of from about 6 to 9, and most desirably from about 7.0 to 7.5. Relatively small

changes in pH radically affect the intensity and special characteristics of fluorescence.

The use of sulfatase enzymes to hydrolyze estriol sulfate conjugates to free estriol is the current method of choice in commercial radioimmunoassays for quantitation of free estriol. The optimal pH for sulfatase enzymes is pH 5.5; thus, the radioimmunoassays utilize a buffer system at this pH to maximize enzyme efficiency. The pH activity profile for sulfatase enzymes falls off sharply at pH 6 with a loss of greater than 95% of its activity at pH 7.3. This nonoverlapping pH optimum for the sulfatase enzyme and the fluorescein tracer precludes their combined use in a homogeneous immunoassay for total estriol.

Various buffers may be used to achieve and maintain the pH of the homogeneous immunoassay of the present invention during the assay procedure. Representative buffers include borate, citrate, acetate phosphate, carbonate, tris, barbital and the like. The particular buffer employed is not critical to the present invention, but the tris and phosphate buffers are preferred. The cation portion of the buffer will generally determine the cation portion of the tracer salt in solution.

The reagents for the fluorescence polarization assay of the present invention comprise antibody specific for estriol and tracer. Additionally, largely conventional solutions including an estriol pretreatment solution, a dilution buffer, estriol calibrators and estriol controls are desirably prepared. Typical solutions of these reagents, some of which are described herein, are commercially available in assay "kits" from Abbott Laboratories, Abbott Park, Illinois.

All percentages expressed herein are weight/volume unless otherwise indicated. The tracer

formulation presently preferred is 70 nanomolar tracer in 0.1 molar tris buffer at pH 5.5, 20% sodium salicylate, and 0.1% sodium azide. The antiserum formulation comprises rabbit serum diluted with 0.1 molar phosphate buffer at pH 7.5, 0.1% sodium azide, 14,000 Fishman units of ß-glucuronidase from E. coli, and 40% sucrose. The dilution buffer comprises 0.1 molar sodium phosphate at pH 7.5, 0.1% sodium azide, and 0.01% bovine gamma globulin. The pretreatment formulation comprises 20% sodium salicylate and 0.1% sodium azide. Calibrators comprising estriol in phosphate-buffered saline at concentrations of 0.0, 30, 60, 105, 210 and 420 ng/ml with 0.1% sodium azide preservative are useful. Controls comprising estriol-16-glucuronide in phosphate-buffered saline are provided at concentrations of 45, 150 and 300 ng/ml with 0.1% sodium azide as a preservative.

The preferred procedure is especially designed to be used in conjunction with the Abbott TDx® polarization analyzer available from Abbott Laboratories, Irving, Texas. Fifty microliters of serum or plasma are required. The calibrators, controls, or unknown samples are pipetted directly into the sample well of the TDx® sample cartridge. One of the advantages of this procedure is that the sample does not require any special preparation. If a TDx® estriol assay kit is being used with the TDx® analyzer, samples are placed directly into a sample carousel, the caps from each of the three reagent containers in the kit are removed and placed into designated wells inside the TDx® analyzer, and the assay procedure from this point is fully automated.

If a manual assay is being performed, then the sample is mixed with the pretreatment solution in dilution buffer and a background reading is taken. The

tracer is then mixed with the assay. The antibody is then finally mixed into the test solution. After incubation, a fluorescence polarization reading is taken.

The fluorescence polarization value of each calibrator, control or sample is determined and is printed on the output tape of an instrument such as the Abbott TDx® polarization analyzer. A standard curve is generated in the instrument by plotting the polarization of each calibrator versus its concentration using nonlinear regression analysis. The concentration of each control or sample is read off the stored calibration curve and printed on the output tape.

With respect to the foregoing preferred procedure, it should be noted that the tracer, antibody, pretreatment solution, calibrators and controls should be stored between about 2 and about 8 degrees C, while the dilution buffer should be stored at ambient temperature. A standard curve and controls should be run every two weeks, with each calibrator and control run in duplicate. Controls should be run daily and all samples can be run in replicates if so desired.

It should be understood that the foregoing detailed description and the following examples are intended to be illustrative, but not limiting, with respect to the scope of the present invention. Various modifications will become apparent to one skilled in the art, and thus it is intended that the scope of the invention be defined solely by the claims and legal equivalents thereof.

## EXAMPLES

### Example 1

This example illustrates the preparation of estriol-3-carboxymethylether-ethylenediamine-5-carboxyfluorescein tracer.

1. Preparation of estriol-3-carboxymethylether:

To estriol (10.0 g) in dry methylethylketone (100 ml) was added potassium carbonate (14.4 g) and ethyl bromoacetate (38.5 ml); the resulting solution was refluxed under an argon atmosphere for 18 hours. Potassium carbonate (14.4 g) was again added, and refluxing continued for another 8 hours. The volatile components were removed under reduced pressure and the residue extracted with methanol. The methanol was removed and the product refluxed in 50% ethanol (100 ml) and 6 N sodium hydroxide hydrochloric acid (20 ml) for 4 hours. The reaction was acidified with 6 N hydrochloric acid to pH 3, and the precipitate was collected and dried under reduced pressure. The yield was 8 g of estriol-3-carboxymethylether (67%) as a white powder.

2. Preparation of estriol-3-carboxymethylether-N-hydroxysuccinimide active ester: Estriol-3-carboxymethylether prepared in step 1 (1.5 g), and N-hydroxysuccinimide (0.60 g) were dissolved in dry N,N-dimethylformamide (10 ml). To this solution was added N,N'-dicyclohexylcarbodiimide (1.08 g) and additional N,N-dimethylformamide (10 ml). The reaction was stirred at room temperature for 18 hours under a nitrogen atmosphere, after which additional N,N'-dicyclohexylcarbodiimide (0.09 g) was added. After 4 hours, the N,N-dimethylformamide was removed under reduced pressure and the residue extracted with 25 ml of ethylacetate to remove N,N'-dicyclohexylurea. The ethyl acetate was removed under reduced pressure and the residue dissolved in methylene chloride (25 ml) and ethyl ether (10 ml). After standing at room temperature for 1 hour, the solution was filtered to remove more N,N'-dicyclohexylurea, and the solvent was then removed under reduced pressure. Ethyl acetate (25 ml) was added to and removed from the residue, causing formation of a clear glass. The

estriol-3-carboxymethylether-N-hydroxysuccinimide active ester was used in the following step without further purification.

3. Preparation of estriol-3-carboxymethylether-ethylenediamine: The active ester prepared in step 2 (0.50 g) in dry N,N-dimethylformamide (7 ml) was added dropwise over 30 minutes to ethylenediamine (1.5 ml) in dry N,N-dimethylformamide (3 ml) cooled in an ice bath. The reaction was stored at 4°C overnight, after which the N,N-dimethylformamide and excess ethylenediamine were removed under reduced pressure. Trace ethylenediamine was removed by coevaporation with methanol:toluene (1:4, 50 ml) three times. The yield was 0.50 g of estriol-3-carboxymethylether-ethylenediamine as a waxy solid.

4. Preparation of 5-carboxyfluorescein-N-hydroxysuccinimide active ester (following the procedure set forth in European Patent Application No. 80300531.3, assigned to Syva Company, Palo Alto, California): To 20.0 g of 5-carboxyfluorescein (obtained from Eastman Kodak Company, Rochester, New York) in dry N,N-dimethylformamide (133 ml) was added N-hydroxysuccinimide (8.2 g). N,N'-dicyclohexylcarbodiimide (12.0 g) was added to this solution and the reaction stirred at room temperature under a nitrogen atmosphere overnight. The N,N-dimethylformamide was removed under reduced pressure and the oil diluted with toluene:acetone:acetic acid (75:23:2) for injection in three portions into a Waters Prep LC/System 500 A liquid chromatograph. The liquid chromatograph was equipped with two silica gel columns and elution was at 100 ml/minute with the aforestated solvent mixture. The chart speed was 2 min./cm, and the detector was on setting 2. Appropriate fractions of the separated 5- and 6-active ester isomers were taken from the three

injections and pooled. Removal of solvent under reduced pressure gave 6.05 g of the 5-isomer (24%), 5.05 g of the 6-isomer (20%), and 5.24 g of the isomer mixture (21%). The product was used in the following step without further characterization.

5. Preparation of estriol-3-carboxymethylether-ethylenediamine-5-carboxyfluorescein tracer: The estriol-3-carboxymethylether-ethylenediamine prepared in step 3 (0.418 g) and the 5-carboxyfluorescein-N-hydroxysuccinimide active ester prepared in step 4 (0.509 g) were dissolved in dry N,N-dimethylformamide (10 ml) under a nitrogen atmosphere at room temperature with stirring, protected from light for 18 hours. The N,N-dimethylformamide was removed under reduced pressure and the residue purified on a Waters Associates Prep LC/System 500 A liquid chromatograph using two 1-inch columns packed with Waters Associates preparative C18 absorbent. Elution was with water:methanol:acetic acid (45:55:05) at 50 ml/minute with a chart speed of 2 min./cm and the detector on setting 5. Appropriate fractions were pooled and the solvent removed under reduced pressure. Traçes of acetic acid were removed by coevaporation with methanol:toluene (1:4, 25 ml) three times. The yield was 0.136 g of estriol-3-carboxymethylether-ethylenediamine-5-carboxyfluorescein (17%), as an orange solid. The tracer is characterized by the formula shown in Figure 9.

Example 2

This example illustrates the preparation of estriol-3-carboxymethylether-4'-N-ethylaminomethylfluorescein tracer in accordance with the teachings of U.S. Patent Application No. ________________, filed concurrently herewith.

To a solution of 0.100 g of estriol-3-carboxymethylether and 0.050 g of N-hydroxysuccinimide in

dry N,N-dimethylformamide was added 0.060 g of N,N'-dicyclohexylcarbodiimide. The solution was stirred at room temperature under an inert atmosphere overnight. This solution was added to 0.123 g of 4'-N-ethylaminomethylfluorescein which had been dissolved in dry N,N-dimethylformamide and 0.060 ml of triethylamine. The reaction was stirred at room temperature under an inert atmosphere overnight, after which the solvent was removed under reduced pressure. The residue was purified by preparative thin-layer chromatography, first with reverse-phase medium (methanol:water:acetic acid), then with silica gel (methanol:methylene chloride:acetic acid, 10:90:0.4), to yield a tracer having the formula shown in Figure 10.

Example 3

This example illustrates the preparation of estriol-3-carboxymethylether-1,3-diaminopropane-5-carboxyfluorescein and estriol-3-carboxymethylether-1,3-diaminopropane-6-carboxyfluorescein tracers.

1. Preparation of estriol-3-carboxymethylether active ester: Estriol-3-carboxymethylether (1.00 g) and N-hydroxysuccinimide (0.398 g) were dissolved in dry N,N-dimethylformamide (10 ml) with stirring. N,N'-dicyclohexylcarbodiimide (0.596 g) was added with additional N,N-dimethylformamide (5 ml) and the reaction stirred at room temperature for 20 hours. The solvent was removed under reduced pressure, the residue dissolved with dry methylene chloride, and the solution filtered. The dissolving and filtering was repeated with methylene chloride and then ethyl acetate, giving a white foam when the solvent was removed under reduced pressure. The yield, after overnight drying under reduced pressure, was 1.45 g of estriol-3-carboxymethylether active ester.

2. Preparation of estriol-3-carboxymethylether-1,3-diaminopropane: To a solution of 1,3-diaminopropane

(0.565 ml) in dry N,N-dimethylformamide (7 ml) was added estriol-3-carboxymethylether active ester (0.300 g) in dry N,N-dimethylformamide at room temperature under an inert atmosphere. After stirring overnight, the solvent and excess 1,3-diaminopropane were removed under reduced pressure.

3. Preparation of the tracers: To the estriol-3-carboxymethylether-1,3-diaminopropane (0.100 g) in 3 ml of dry N,N-dimethylformamide was added the carboxy fluorescein active ester mixture (0.118 g) as a solid. The reaction was stirred at room temperature under an inert atmosphere overnight, and the solvent was removed under reduced pressure. The residue was purified using preparative reverse-phase thin-layer chromatography, yielding the isolated estriol-3-carboxymethylether-1,3-diaminopropane-5-carboxyfluorescein and estriol-3-carboxymethylether-1,3-diaminopropane-6-carboxyfluorescein tracers: The formulae are shown in Examples 11 and 12, respectively.

## Example 4

This example illustrates the preparation of estriol-3-carboxymethylether-piperazine-5-carboxyfluorescein and estriol-3-carboxymethylether-piperazine-6-carboxyfluorescein tracers.

1. Estriol-3-carboxymethylether active ester was prepared as previously described.

2. Preparation of estriol-3-carboxymethylether-piperazine: To a solution of piperazine (0.583 g) in dry N,N-dimethylformamide (7 ml) was added 0.300 g of the carboxymethylether active ester and 3 ml of additional dry N,N-dimethylformamide. The reaction was stirred at room temperature overnight, and the solvent was then removed under reduced pressure. The residue was triturated with ethyl acetate and dried under reduced pressure.

3. Preparation of the tracers: To a solution of the estriol-3-carboxymethylether-piperazine (0.090 g) in dry N,N-dimethylformamide (2 ml) was added 0.103 g of carboxyfluorescein active esters. The reaction was stirred at room temperature under an inert atmosphere overnight, and the solvent was then removed. The residue was purified by preparative thin-layer chromatography using reverse-phase medium. This yielded the estriol-3-carboxymethylether-piperazine-5-carboxyfluorescein and estriol-3-carboxymethylether-piperazine-6-carboxyfluorescein tracers shown in Figures 13 and 14, respectively.

Example 5

This example illustrates the preparation of estriol-3-carboxymethylether-N,N'-dimethylethylenediamine-5-carboxyfluorescein and estriol-3-carboxymethylether-N,N'-dimethylethylenediamine-6-carboxyfluorescein tracers.

1. The preparation of estriol-3-carboxymethylether active ester was previously described.

2. Preparation of estriol-3-carboxymethylether-N,N'-dimethylethylenediamine: To a solution of the estriol-3-carboxymethyether active ester (0.300 g) in dry N,N-dimethylformamide (2 ml) were added dimethylethylenediamine (0.720 ml). The reaction was stirred at room temperature under an inert atmosphere overnight. The solvent and excess diamine were then removed under reduced pressure and the residue triturated with ethyl acetate and then dried.

3. Preparation of the tracers: To a solution of estriol-3-carboxymethylether-N,N'dimethylethylenediamine (0.090 g) in dry N,N-dimethylformamide (2 ml) was added the carboxyfluorescein active esters (0.102 g). The reaction was stirred at room temperature under an inert atmosphere overnight. The solvent was then removed under

reduced pressure and the residue purified by preparative thin-layer chromatography, using reverse-phase medium, to provide estriol-3-carboxymethylether-N,N'-dimethylethylenediamine-5-carboxyfluorescein and estriol-3-carboxymethylether-N-N'-dimethylethylenediamine-6-carboxyfluorescein tracers shown in Figures 15 and 16 respectively.

Example 6

This example illustrates the preparation of estriol-6-carboxymethyloxime-4'-aminomethylfluorescein tracer.

To a solution of estriol-6-carboxymethyloxime (0.0412 g) and N-hydroxysuccinimide (0.0152 g) in dry N,N-dimethylformamide (1 ml) was added N,N-dicyclohexylcarbodiimide (0.023 g) and additional dry N,N-dimethylformamide (0.5 ml). After 20 hours at room temperature and filtering to remove N,N-dicyclohexylurea, this active ester was added to 4'-aminomethylfluorescein (0.046 g) which had been dissolved in dry N,N-dimethylformamide (0.30 ml) and triethylamine (0.046 ml). After 20 hours at room temperature, the solvent was removed and the residue purified by preparative thin-layer chromatography with reverse-phase medium using methanol:water:acetic acid (70:30:0.5) to yield the tracer shown in Figure 14.

Example 7

This example illustrates the preparation of estriol-6-carboxymethyloxime-4'-N-methylaminomethylfluorescein tracer shown in Figure 15.

To a solution of estriol-6-carboxymethyloxime (0.027 g) and N-hydroxysuccinimide (0.010 g) in dry N,N-dimethylformamide (1 ml) was added N,N'-dicyclohexylcarbodiimide (0.015 g) and additional N,N-dimethylformamide (0.5 ml). After 20 hours at room temperature, the solution was filtered through

diatomaceous earth into a solution of 4'-N-methylaminomethylfluorescein (0.031 g) and triethylamine (0.030 ml) in dry N,N-dimethylformamide (0.5 ml). After 20 hours at room temperature and sealed from air, the solvent was removed under reduced pressure and the residue purified by preparative thin-layer chromatography with reverse-phase medium using methanol:water:acetic acid (70:30:0.5) to yield the tracer shown in Figure 18.

<u>Example 8</u>

This example illustrates the preparation of estriol-6-carboxymethyloxime-4'-N-n-butylaminomethylfluorescein tracer.

To a solution of estriol-6-carboxymethyloxime (0.200 g) and N-hydroxysuccinimide (0.123 g) in dry N,N-dimethylformamide (2 ml) was added N,N'-dicyclohexylcarbodiimide (0.132 g) and additional N,N-dimethylformamide (1 ml). After 20 hours at room temperature under an inert atmosphere, the solution was filtered through diatomaceous earth into a solution of 4'-N-butylaminomethylfluorescein (0.242 g) and triethylamine (0.223 ml) in dry N,N-dimethylformamide (3 ml). After 20 hours at room temperature under an inert atmosphere and protected from light, the solvent was removed under reduced pressure and the residue purified by preparative thin-layer chromatography, first with silica gel and then with reverse-phase medium to yield the tracer shown in Figure 19.

<u>Example 9</u>

This example illustrates the preparation of estriol-6-carboxymethyloxime-piperazine-carboxyfluorescein tracers.

1. Preparation of carboxyfluorescein-piperazine: To a solution of piperazine (3.6 g) in dry N,N-dimethylformamide (10 ml)

was added carboxyfluorescein active ester (1.00 g) in dry N,N-dimethylformamide (5 ml) dropwise over 10 minutes. After 18 hours at room temperature under an inert atmosphere, the solvent was removed under reduced pressure and the residue triturated, first with ethyl acetate and then with n-butanol, until it became a fine orange powder.

2. Preparation of the tracers: To a solution of estriol-6-carboxymethyloxime (0.050 g) and N-hydroxysuccinimide (0.018 g) in dry N,N-dimethylformamide (1 ml) was added N,N'-dicyclohexylcarbodiimide (0.028 g). After 20 hours at room temperature under an inert atmosphere, the active ester was filtered and added to a solution of the carboxyfluorescein-piperazine (0.059 g) dissolved in dry N,N-dimethylformamide (1.0 ml). After 20 hours at room temperature under an inert atmosphere, the solvent was removed under reduced pressure and the residue purified by preparative thin-layer chromatography with reverse-phase medium using methanol:water:acetic acid to yield estriol-6-carboxymethyloxime-piperazine-5-carboxyfluorescein and estriol-6-carboxymethyloxime-piperazine-6-carboxyfluorescein tracers. The structural formulae for these tracers are shown in Figures 20 and 21, respectively.

## Example 10

This example illustrates the preparation of estriol-6-carboxymethyloxime-N,N'-dimethylethylenediamine-carboxyfluorescein tracers.

Preparation of carboxyfluorescein-N,N'-dimethylethylenediamine: To a solution of N,N'-dimethylethylenediamine (2.25 ml) in N,N-dimethylformamide (7 ml) was added carboxyfluorescein active ester (1.00 g) in dry N,N-dimethylformamide (8 ml) dropwise over 10 minutes. The reaction was stirred at room temperature under an inert atmosphere for 4 hours. The solvent was then removed under reduced pressure and

the residue triturated with ethyl acetate several times and then dried.

2. Preparation of the tracers: To a solution of estriol-3-carboxymethyloxime (0.068 g) and N-hydroxysuccinimide (0.025 g) in dry N,N-dimethylformamide (2 ml) was added N,N'-dicyclohexylcarbodiimide (0.038 g). After being stirred at room temperature under an inert atmosphere for 20 hours, the solution was filtered and added to a solution of carboxyfluorescein-N,N'-dimethylethylenediamine (0.081 g) in dry N,N-dimethylformamide (1 ml) and stirred for another 20 hours at room temperature under an inert atmosphere. The solvent was removed under reduced pressure and the residue purified by preparative thin-layer chromatography with reverse-phase medium using methanol:water:acetic acid to yield the estriol-6-carboxymethyloxime-N,N'-dimethylethylenediamine-5-carboxyfluorescein and estriol-6-carboxymethyloxime-N,N'-dimethylethylenediamine-6-carboxyfluorescein tracers shown in Figures 22 and 23, respectively.

Example 11

This example illustrates the preparation of estriol-6-carboxymethyloxime-N,N'-diethylethylenediamine-carboxyfluorescein tracers.

1. Preparation of carboxyfluorescein-N,N'-diethylethylenediamine: To a solution of N,N'-diethylethylenediamine (6.0 ml) in dry N,N-dimethylformamide (10.0 ml) was added carboxyfluorescein active ester (1.00 g) in dry N,N-dimethylformamide (5.0 ml) dropwise over 10 minutes. The solution was stirred overnight at room temperature. The solvent was removed under reduced pressure and the residue triturated with ethyl ether and then ethyl acetate until the product became a fine powder.

2. Preparation of the tracers: To a solution of estriol-3-carboxyethyloxime (0.050 g) and N-hydroxysuccinimide (0.018 g) in dry N,N-dimethylformamide (1 ml) was added N,N'-dicyclohexylcarbodiimide (0.028 g). After 20 hours at room temperature under an inert atmosphere, the active ester was filtered and added to a solution of carboxyfluorescein-N,N'-diethylethylenediamine (0.060 g) in dry N,N-dimethylformamide (1 ml). After 20 hours at room temperature under an inert atmosphere, the solvent was removed under reduced pressure and the residue purified by preparative thin-layer chromatography with reverse-phase medium using methanol:water:acetic acid to yield estriol-6-carboxymethyloxime-N,N'-diethylethylenediamine-5-carboxyfluorescein and estriol-6-carboxymethyloximine-N,N'-diethylethylenediamine-6-carboxyfluorescein tracers. These tracers are shown in Figures 24 and 25, respectively.

Example 12

This example illustrates the preparation of estriol-6-carboxymethyloxime-ethylenediamine-carboxyfluorescein tracers.

1. Preparation of carboxyfluorescein-1,2-ethylenediamine: To a solution of ethylenediamine (0.60 ml) in dry N,N-dimethylformamide (5.0 ml) was added a solution of carboxyfluorescein active esters (0.40 g) in dry N,N-dimethylformamide (4.0 ml) dropwise and with stirring under an inert atmosphere. After 2 hours the solvent was removed under reduced pressure and the residue triturated with ethyl acetate and dried under reduced pressure.

2. Preparation of the tracers: To a solution of estriol-3-carboxymethyloxime (0.030 g) and N-hydroxysuccinimide (0.012 g) in dry

N,N-dimethylformamide (0.50 ml) was added N,N'-dicyclohexylcarbodiimide (0.025 g). After 20 hours at room temperature the carboxyfluorescein-ethylenediamine (0.033 g) was dissolved in dry N,N-dimethylformamide (0.50 ml) and glacial acetic acid (0.10 ml). The acetic acid was neutralized with triethylamine and then the carboxyfluorescein-ethylenediamine solution was added to the estriol-6-carboxymethyloxime active ester solution. After 20 hours the solvent was removed under reduced pressure and the residue purified by preparative thin-layer chromatography with reverse-phase medium using methanol:water:acetic acid to yield estriol-6-carboxymethyloxime-ethylenediamine-5-carboxyfluorescein and estriol-6-carboxymethyloxime-ethylenediamine-6-carboxyfluorescein tracers. These tracers are shown in Figures 26 and 27, respectively.

Example 13

This example illustrates the preparation of estriol-7-carboxyethylthioether, which serves as an intermediate in the preparation of the tracers in Examples 14, 15, 16 and 17.

1. Preparation of estriol triacetate: To a stirred suspension of estriol (47.1 g), acetic anhydride (160 ml) and pyridine (160 ml) was added 0.98 g of 4-N,N-dimethylaminopyridine. A solution was obtained in 5 minutes. After stirring at room temperature for 1 hour, the reagents were removed *in vacuo* at room temperature to give a gummy residue. The triacetate was dissolved in ethyl acetate (500 ml), shaken with water (4 x 500 ml) and dried over sodium sulfate. Removal of the dried solvent gave 65.87 g (97%) of estriol triacetate.

2. Preparation of 6-ketoestriol triacetate: To a cold (0°C), mechanically stirred solution of dry pyridine (162 ml) in dry methylene chloride (700 ml) was rapidly added chromium trioxide (97.2 g). After stirring

for 15 minutes, a solution of estriol triacetate (68 g) in methylene chloride (300 ml) was added. The reaction mixture was mechanically stirred at room temperature for 36 hours. The dark mixture was then filtered through a short column of Florisil[R] (200 g), washing carefully with diethyl ether (1 l). The combined filtrates were concentrated in vacuo to yield a gummy solid, which was purified by elution from silica (300 g) using 9:1 hexane:ethyl acetate to give 31.7 g of unchanged starting material. Continued elution gave 18.4 g of the 6-keto compound. The product was further purified by crystallization from diethyl ether to yield 13.0 g (36% based on recovered starting material) of pure 6-ketoestriol triacetate.

3. Preparation of 6-dehydroestriol triacetate: To a cold (0°) solution of the 6-keto compound (11.5 g) in tetrahydrofuran (200 ml) and methanol (400 ml) was cautiously added, in portions, sodium borohydride (7.5 g), followed by the addition of 15 ml of water. After stirring for 45 minutes, the reaction was quenched by the addition of acetic acid (12 ml). The solvents were removed in vacuo and the residue dissolved in ethyl acetate (1 l) and washed with water (3 x 200 ml). The solution was dried over sodium sulfate. Removal of the dried solvent in vacuo gave 10.2 g of the 6-hydroxy compound as a semicrystalline solid. The crude 6-hydroxy compound was dissolved in methanol (400 ml) and treated with 100 ml of 12 N hydrochloric acid. After stirring at 60°C for 2 hours, the reaction mixture was cooled and concentrated in vacuo. The residue was diluted with water (500 ml), extracted with ethyl acetate (2 x 500 ml) and dried over sodium sulfate. Removal of the dried solvent in vacuo yielded 5.9 g of crystals. This material was dissolved in pyridine (20 ml) and acetic anhydride (20 ml) and treated with 4-N,N-dimethylaminopyridine (150 mg).

After stirring at room temperature for 1 hour, the excess reagents were removed *in vacuo* at 30°C. The red residue was dissolved in ethyl acetate (500 ml), washed with water (3 x 200 ml) and dried over sodium sulfate. Removal of the dried solvent *in vacuo* gave 8.3 g of red crystals, which were triturated with diethyl ether (25 ml) to yield 3.9 g of light-pink crystals (mp 150°-153°C). Additional product was obtained by elution of the mother liquors from silica gel (100 g) with ethyl acetate:hexane (1:4) to give 1.95 g of colorless crystals for a total yield of 5.85 g (53%).

4. Preparation of 7 -(2-methoxycarbonylethylmercapto)estriol triacetate:

To a solution of the tetraene (5.5 g, 13.3 mmol) in methyl 3-mercaptopropionate (25 ml) was added 20 mg of 2,2$^1$-azobisisobutyronitrile. The 50 ml round bottom flask was swept with nitrogen, stoppered and placed 5 cm from an Hanovia high pressure quartz mercury lamp (450 watts) and irradiated for 1 hour. The excess reagent was removed *in vacuo* (0.5 mm Hg) at 40°C to give an oil. The product was purified by elution from silica gel (200 g) using ethyl acetate hexane (1:5), to give 5.3 g (75%) of 7 -(2-methoxycarbonylethylmercapto)estriol triacetate as a mixture of epimers at C-7.

5. Preparation of 7 -(carboxyethylmercapto)estriol:

To a solution of the above triacetate methyl ester (500 mg) in methanol (20 ml) was added 2 N potassium hydroxide (5 ml). After stirring at room temperature for 1 hour, the reaction mixture was cooled (0°) and acidified to pH 3 with 12 N hydrochloric acid. The mixture was diluted with toluene and the solvents removed *in vacuo*. The resultant gum was thoroughly washed with water to remove potassium chloride and the gum dried by azeotroping

several times _in vacuo_ with toluene to give 315 mg (86%) of estriol-7-carboxyethylthioether as a foam.

Example 14

This example illustrates the preparation of 7-(2-carboxyethylmercapto) estriol-4'-aminomethylfluorescein tracer. A solution of 7-(2-carboxyethylmercapto) estriol (9:1 :ß-isomeric mixture (20 mg), 1-ethyl-3-(3-dimethylaminopropyl) carbodiimide (10 mg) and N-hydroxysuccinimide (7.5 mg) in dry methylene chloride (0.5 ml) was stirred for 18 hours under anhydrous conditions. After this period, anhydrous N,N-dimethylformamide (0.05 ml) was added and the stirring continued for 24 hours. To this solution 4'-aminomethylfluorescein (20 mg) was added and the stirring continued for another 24 hours. After basification with triethylamine, the reaction mixture was stirred for 18 hours and the tracer was purified _via_ silica gel (methanol in methylene chloride) and reverse phase (65:35:0.5 methanol:water:acetic acid) thin-layer chromatography. This tracer is shown in Figure 28.

Example 15

This example illustrates the preparation of 7-(2-carboxyethylmercapto) estriol-4'-N-ethylaminomethylfluorescein tracer. A solution of 7-(2-carboxyethylmercapto) estriol (9:1 :ß isomeric mixture, (20 mg), 1-ethyl-3-(3-dimethylaminopropyl) carbodiimide (10 mg) and N-hydroxysuccinimide (7.5 mg) in dry N,N-dimethylformamide (0.4 ml) was stirred, protected from moisture for 18 hours. After the addition of 4'-N-ethylaminomethylfluorescein (20 mg), the reaction mixture was basified with triethylamine and stirred for another 18 hours. Addition of diethyl ether gave a precipitate of crude tracer which was purified _via_ thin

layer chromatography on silica gel (10.1 $CH_3OH$ in $CH_2Cl_2$). The structural formula is shown in Figure 29.

Example 16

This example illustrates the preparation of 7-(2-carboxyethylmercapto) estriol-N,N'-dimethylethylenediamine-5-carboxyfluorescein and 7-(2-carboxyethylmercapto) estriol-N,N'-dimethylethylenediamine-6-carboxyfluorescein tracers. Active ester prepared as in Example 15 (20 mg) was allowed to react with a mixture of 5-, and 6-carboxyfluorescein-N,N'-dimethylethylenediamine (20 mg) in the presence of a catalytic amount of triethylamine for 18 hours. The resulting tracers were separated and purified via thin-layer chromatography on reverse phase (60:40:0.5 methanol:water:acetic acid). The 5- and 6-carboxyfluorescein tracers are shown in Figures 30 and 31, respectively.

Example 17

This example illustrates the preparation of 7-(2-carboxyethylmercapto)estriol-bovine serum albumin immunogen. A solution of 7-(2-carboxyethylmercapto) estriol (30 mg), 1-ethyl-(3-dimethylaminopropyl) carbodiimide hydrochloride (18 mg) and N-hydroxysuccinimide (15 mg) was stirred, protected from moisture in dry N,N-dimethylformamide (0.5 ml) for approximately 6 hours. This reaction mixture was added dropwise with vigorous stirring to a solution of bovine serum albumin (70 mg) in borate buffer (0.5 ml, 0.1 M, pH8). After 18 hours, it was diluted with water (5 ml) and dialyzed against a solution of sodium bicarbonate (1%) followed by distilled water. The slurry upon lyophylization gave a white powder (67 mg). The structural formula of the immunogen is shown in Figure 32.

Example 18

This example illustrates one preferred immunoassay embodiment.

Estriol-3-carboxymethylether-ethylenediamine-5-carboxyfluorescein was synthesized as described in Example 1. Ten mg of this tracer was dissolved in 20 ml of ethanol. Three µl of the resulting stock tracer solution was diluted into 10 ml of 20% sodium solicylate to give a tracer concentration of 70 nanomolar.

Antiserum 16 (Table I) was diluted in 100 ml of phosphate buffer (pH 6.2) containing 40% sucrose and 14,000 modified Fishman units of E. coli ß-glucuronidase (obtained from Sigma Corporation, St. Louis, Missouri) to hydrolyze the estriol glucuronide. (One Sigma or modified Fishman unit will liberate 1.0 g of phenolphthalein from phenolphthalein glucuronide per hour at 37° C in phosphate buffer, pH 6.8).

25 µl of antiserum reagent 16 and 7 µl of sample were mixed together with 500 µl of dilution buffer comprising 0.1 molar sodium phosphate at pH 7.5, 0.1% sodium azide, and 0.01% bovine gamma globulin (pH 7.3) in the predilution well of an Abbott TDx® polarization analyzer. 175 µl of this prediluted sample and antiserum mixture was then transferred to a cuvette together with 25 µl of 20% sodium salicylate and 925 µl of the aforementioned dilution buffer. After a 10-minute incubation period, a fluorescent sample background reading was taken.

175 µl of prediluted sample was next transferred into a cuvette together with 25 µl of the estriol-3-carboxymethylether-ethylenediamine-5-carboxyfluorescein tracer and 925 µl of the dilution buffer. After a 4-minute incubation period, a final reading is taken and estriol concentration values are printed off a stored standard curve.

The results are shown below in Graph I. A binding inhibition standard curve was the same for estriol and estriol-3-sulfate, demonstrating the ability of the assay to measure estriol-3-sulfate without enzyme hydrolysis thereof.

GRAPH I

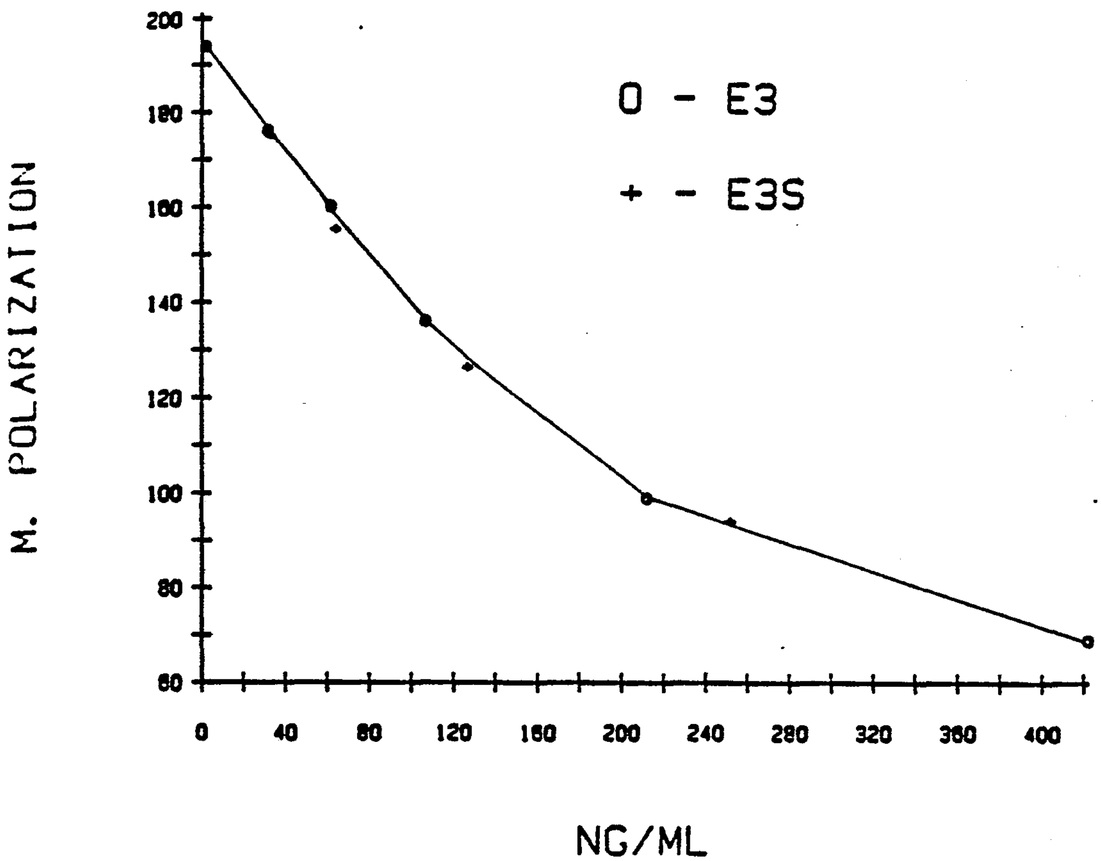


Example 19

Example 18 was repeated, with substitution of the estriol-6-carboxymethyloxime-4'-N-ethylaminomethylfluorescein tracer prepared in Example 6 for the estriol-3-carboxymethylether-ethylenediamine-5-carboxyfluorescein tracer and substitution of antiserum 12 for antiserum 16. As Table I indicates, this combination also yielded 100%

cross-reactivity.

Example 20

Example 18 was repeated with substitution of antiserum 10 for antiserum 16. As Table I indicates, this combination also yielded 100% cross-reactivity.

Example 21

Example 18 was repeated except that a pool of antisera from four rabbits (No. 2, 10, 13, 16), each having estriol-3-sulfate cross-reactivity less than, equal to, or greater than 100%, was substituted for antiserum 16. The pooling procedures was as follows: Antiserum having less than 100% cross-reactivity (rabbit No. 2) was combined with antiserum having greater than 100% cross-reactivity (rabbit No. 13) until the combined estriol-3-sulfate cross-reactivity was equal to 100%. This pool of antisera was then combined with a pool of antisera that originally had 100% cross-reactivity (rabbit Nos. 10 and 16).

Preparation of a binding inhibition standard curve, similar to that shown in Example 18, showed the same inhibition for both estriol and estriol-3-sulfate.

The accuracy of the assay was also verified by correlation with other assays. Both serum and urine specimens covering a broad range of total estriol values were used. The total estriol fluorescence polarization immunoassay of the present invention was compared with the total estriol/radioimmunoassay of Amersham International PLC (England) and the GammaDab® total estriol radioimmunoassay of Clinical Assays (Baxter Travenol, Deerfield, Illinois). Seventy-five urine specimens were also evaluated by high-performance liquid chromatography (HPLC). The results are summarized in Table V.

TABLE V

| | TDx Total Estriol vs. | Number of Observations | Inter-cept | Slope | Correlation Coefficient |
|---|---|---|---|---|---|
| Serum | Amersham Total Estriol RIA | 154 | -1.74 | 1.06 | 0.993 |
| | Clinical Assays GammaDab® [$^{125}$ I) Total Estriol | 148 | 4.4 | 0.96 | 0.991 |
| Urine | Amersham Total Estriol RIA | 153 | -1.62 | 1.11 | 0.992 |
| | Clinical Assays GammaDab® [$^{125}$ I) Total Estriol | 151 | 0.97 | 1.00 | 0.981 |
| | HPLC | 75 | -0.49 | 1.00 | 0.962 |

CLAIMS

1. A compound comprising the structure:

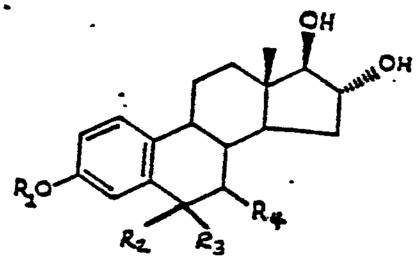

wherein $R_1$ is H or R-Z-Q; $R_2$ and $R_3$ are H when $R_1$ is R-Z-Q and are taken together as R-Z-Q when $R_1$ and $R_4$ are H; $R_4$ is R-Z-Q when $R_1$, $R_2$ and $R_3$ are H and is H when either $R_1$ or $R_2$ taken together with $R_3$ is R-Z-Q.

Q is a poly (amino acid), a poly (amino acid) derivative or another immunologically active carrier, or fluorescein or a fluorescein derivative;

Z is CO, NH, $CH_2NH$ or CS when Q is fluorescein or a fluorescein derivative and is N, NH, $SO_2$, $PO_2$, PSO or a glucuronide moiety when Q is a poly (amino acid), a poly (amino acid) derivative or another immunologically active carrier; and

R is a linking group including up to 7 heteroatoms when Q is a poly (amino acid), a poly (amino acid) derivative or other immunologically active carrier and including up to 10 heteroatoms when Q is fluorescein or a fluorescein derivative, and having a total of from 0 to 20 carbon atoms and heteroatoms arranged in a straight or branched chain and containing up to two ring structures.

2. The compound of claim 1 wherein Q is bovine serum albumin.

3. The compound of claim 1 wherein Q is a carboxyfluorescein, 4'-aminomethylfluorescein or fluoresceinamine derivative.

4. An antibody to a compound according to claim 1 wherein Q is a poly (amino acid) or a poly (amino acid) derivative or another immunologically active carrier.

5. A process for making an immunogen comprising the step of coupling a precursor of the formula:

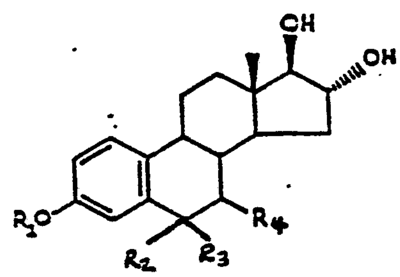

wherein...

$R_1$ is H or R-X; $R_2$ and $R_3$ are H when $R_1$ is R-X and are taken together as R-X when $R_1$ and $R_4$ are H; $R_4$ is R-X when $R_1$, $R_2$ and $R_3$ are H and is H when either $R_1$ or $R_2$ taken together with $R_3$ is R-X;

X is $NH_2$, $SO_3H$, $PO_3H$, $PSO_2H$ or a glucuronide moiety when $R_2$, $R_3$ and $R_4$ are H, is $NH_2$ or COOH when $R_1$, $R_2$ and $R_3$ are H and is $NH_2$ when $R_1$ and $R_4$ are H; and

R is a linking group including up to 7 heteroatoms and having a total of from 0 to 20 carbon atoms and heteroatoms arranged in a straight or branched chain and containing up to two ring structures

with a poly (amino acid), a poly (amino acid) derivative or another immunologically active carrier.

6. The method of claim 5 wherein the poly (amino acid) is bovine serum albumin.

7. The method of claim 5 wherein the reaction is carried out by water-soluble carbodiimide coupling.

8. A method of making a tracer comprising the step of coupling a precursor of the formula

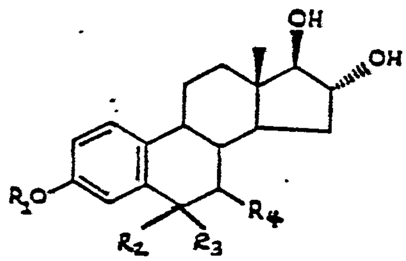

wherein $R_1$ is H or R-Y; $R_2$ and $R_3$ are H when $R_1$ is R-Y and are taken together as R-Y when $R_1$ and $R_4$ are H; $R_4$ is R-Y when $R_1$, $R_2$ and $R_3$ are H and is H when either $R_1$ or $R_2$ taken together with $R_3$ is R-Y;

Y is $NH_2$, COOH, $SO_3H$, $SO_2Cl$, SH, CHO, CN, OH or a glucuronide moiety; and

R is a linking group including up to 10 heteroatoms and having a total of from 0 to 20 carbon atoms and heteroatoms arranged in a straight or branched chain and containing up to two ring structures

with fluorescein or a derivative of fluorescein.

9. A process for measuring the total estriol content of a fluid sample, which comprises the steps of:

(a) contacting the fluid sample with an estriol antiserum, a ß-glucuronidase enzyme, and a compound of claim 1 capable of producing a detectable fluorescence polarization response to the presence of the estriol antiserum;

(b) passing plane polarized light through the resulting solution from step (a) to obtain a fluorescence polarization response; and

(c) detecting the fluorescence polarization response of the solution of step (b) as a measure of the total estriol content of the sample.

10. A reagent system for use in conducting a fluorescence polarization immunoassay comprising:

(a) a compound according to claim 3; and

(b) an antibody having a cross-reactivity with estriol, estriol-3-sulfate and the compound of claim 3 of about 90% to about 110%.

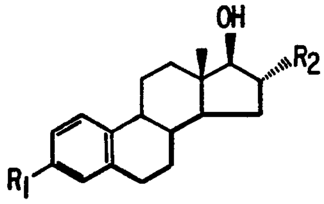

FIG.1

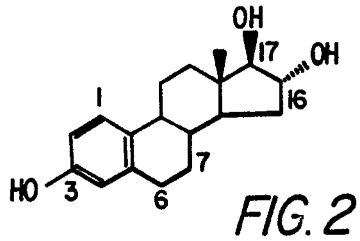

FIG. 2

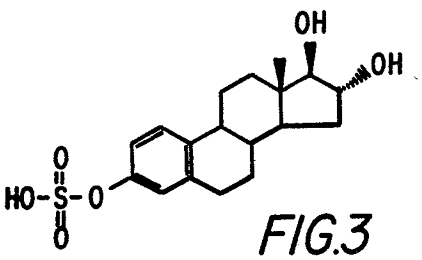

FIG.3

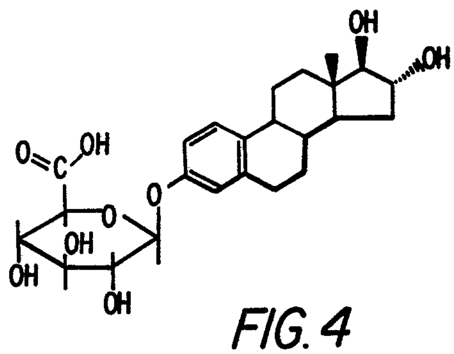

FIG. 4

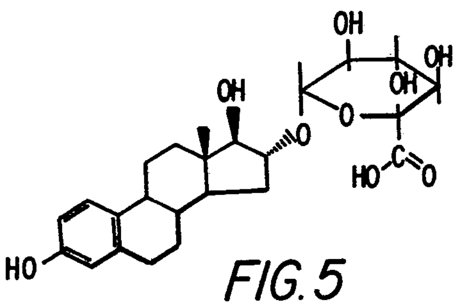

FIG. 5

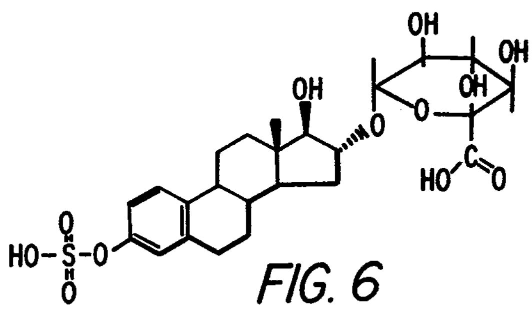

FIG. 6

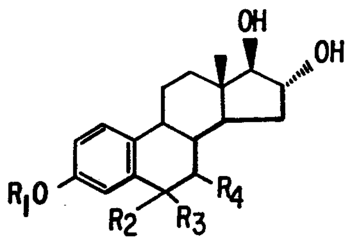

FIG.7

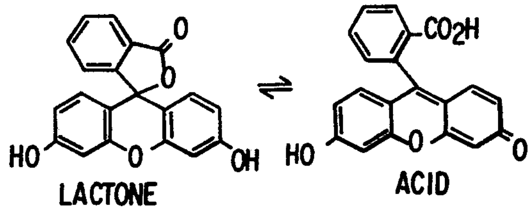


FIG.8

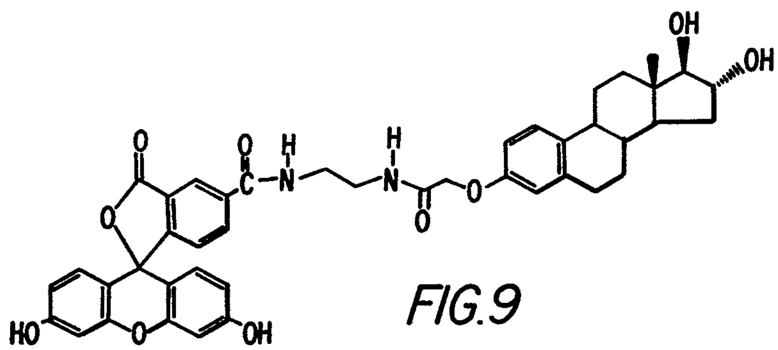

FIG.9

FIG.10

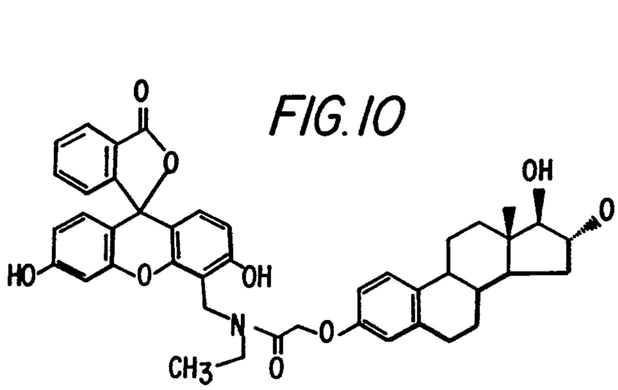

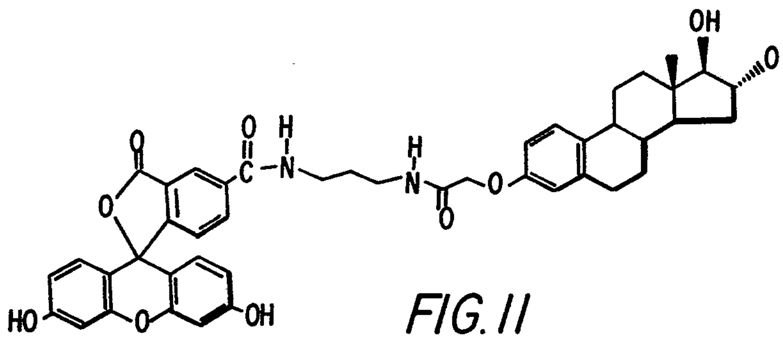

FIG. 11

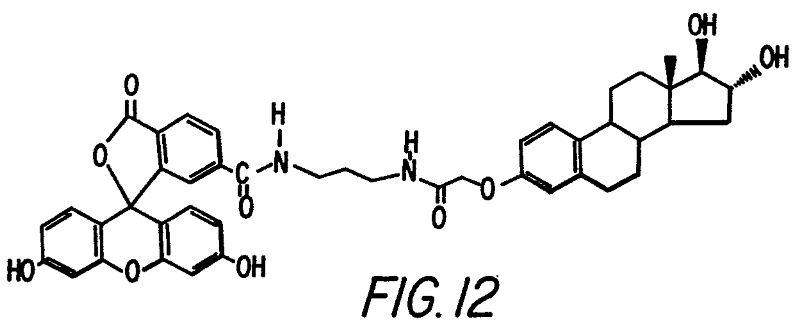

FIG. 12

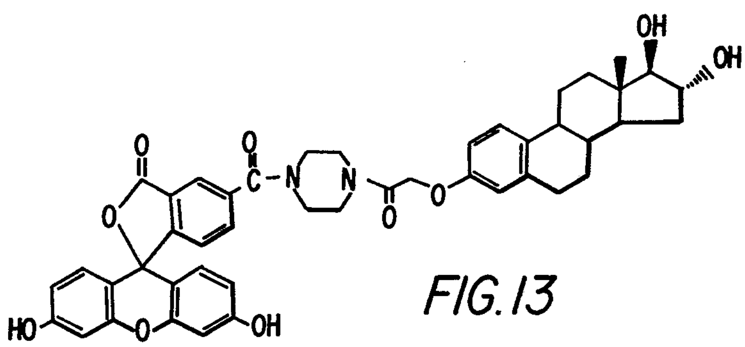

FIG. 13

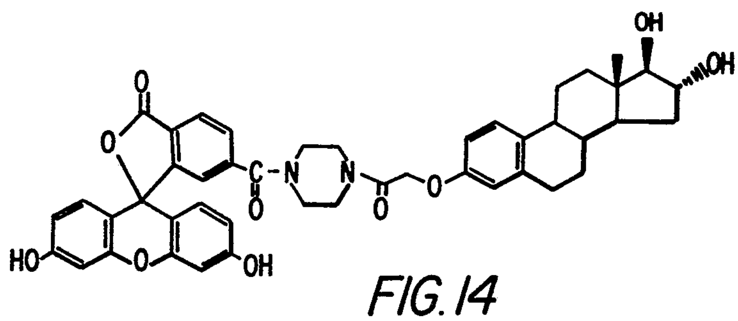

FIG. 14

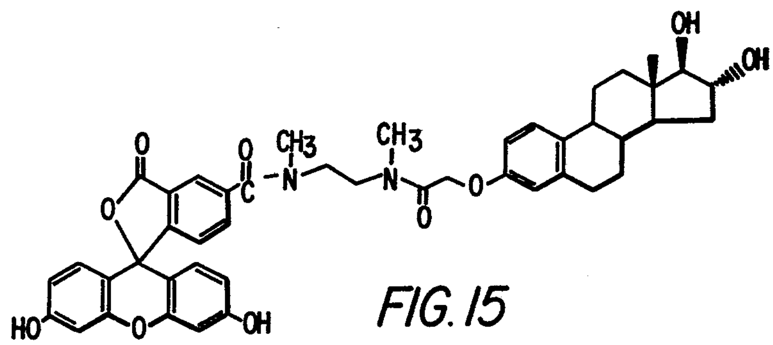

FIG. 15

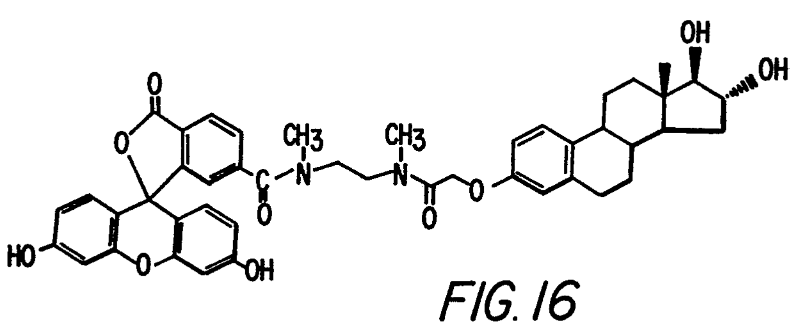

FIG. 16

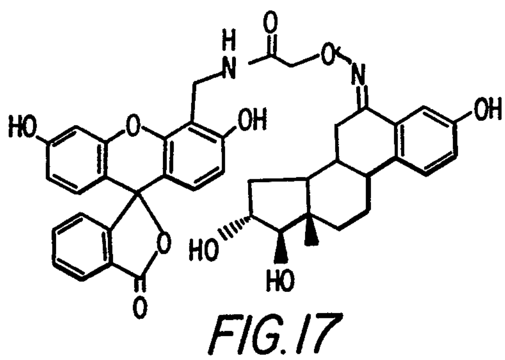

FIG. 17

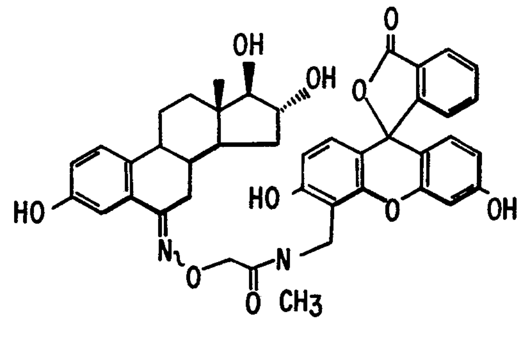

FIG. 18

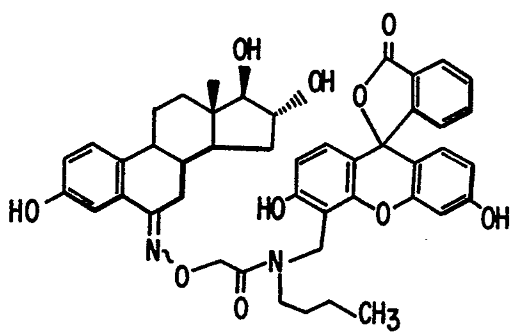

FIG. 19

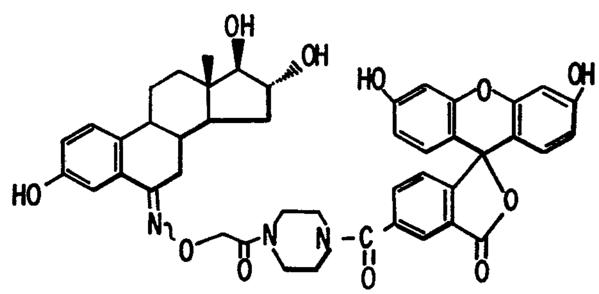

FIG. 20

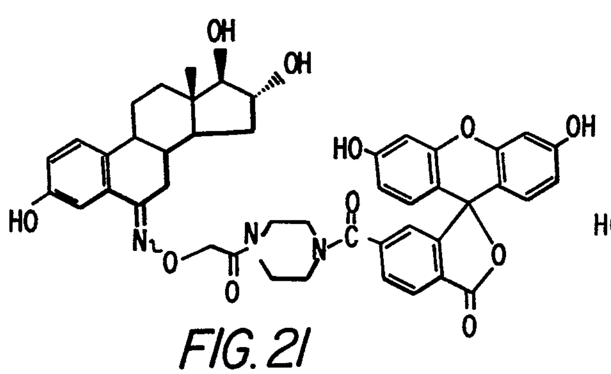

FIG. 21

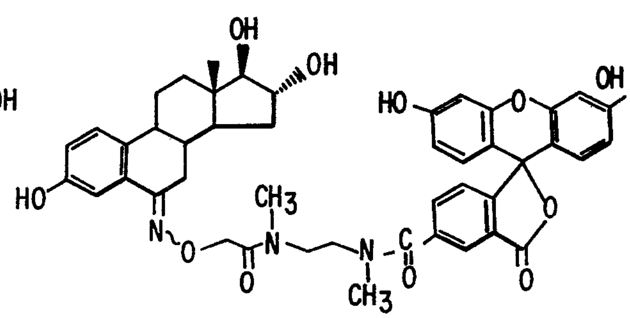

FIG. 22

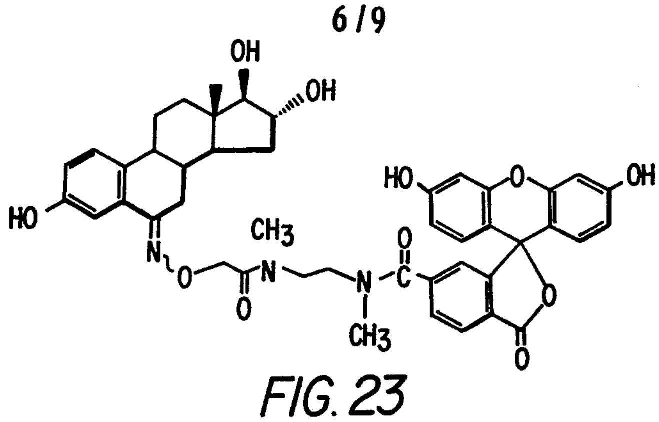

FIG. 23

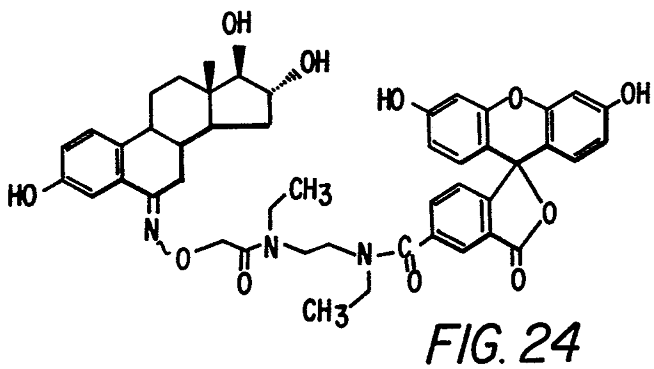

FIG. 24

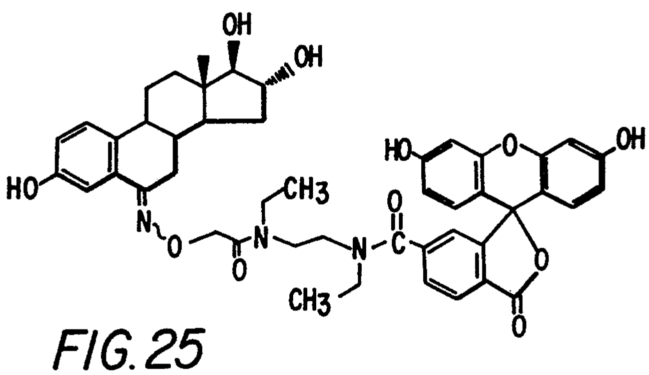

FIG. 25

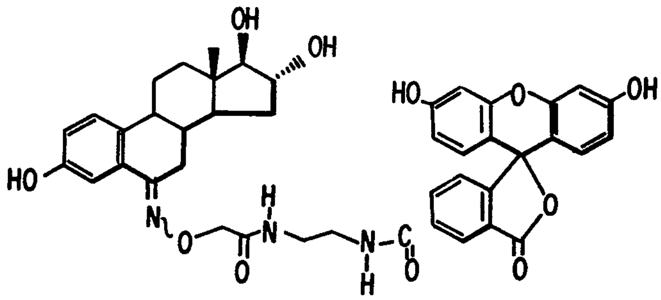

FIG. 26

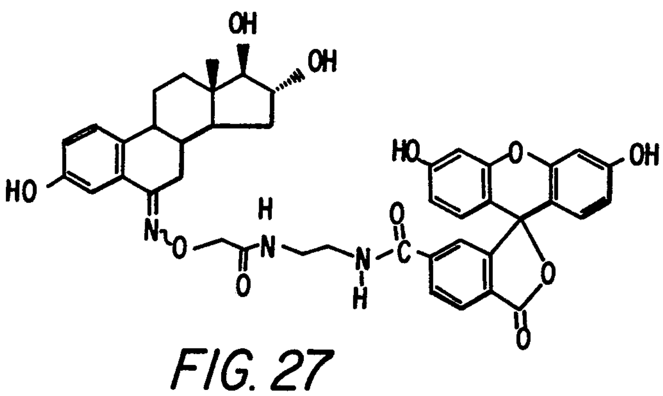

FIG. 27

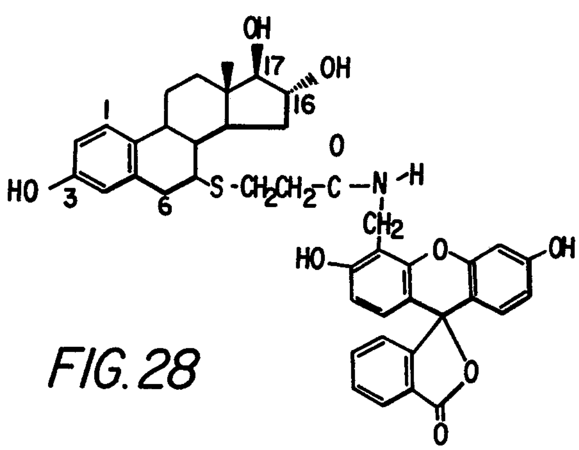

FIG. 28

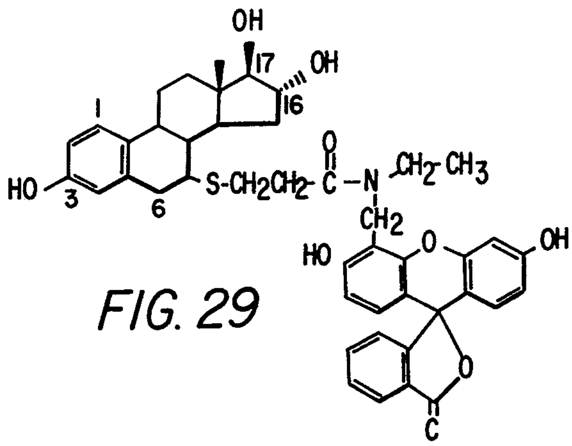

FIG. 29

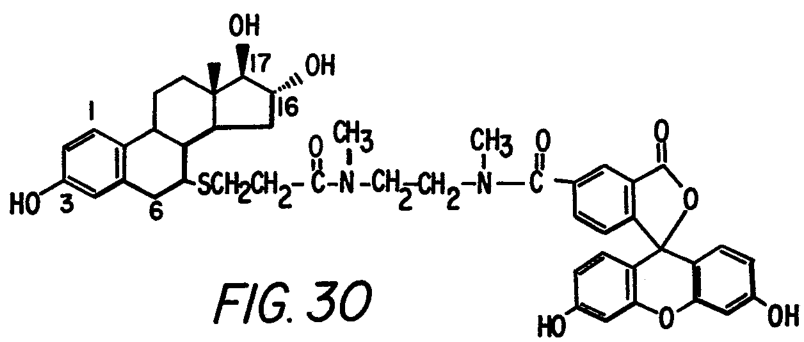

FIG. 30

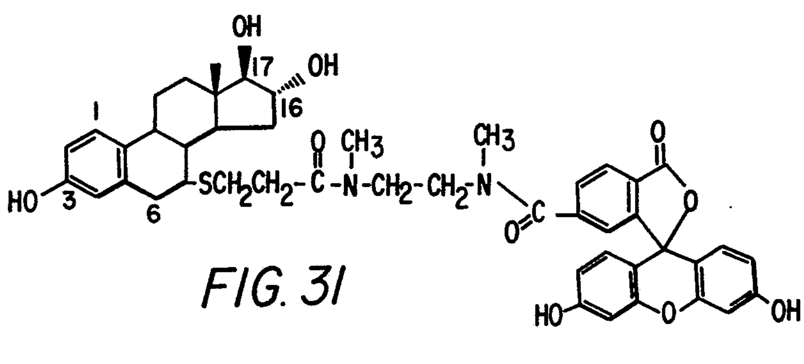

FIG. 31

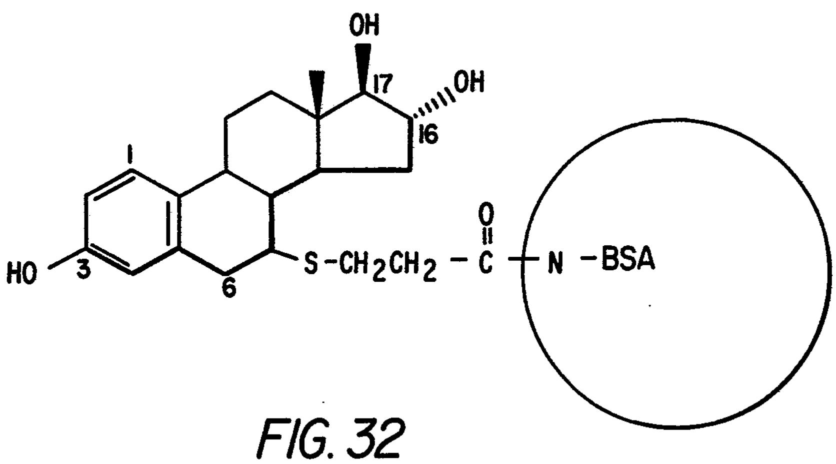

FIG. 32

European Patent Office

# EUROPEAN SEARCH REPORT

0200960

Application number

EP 86105153.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US - A - 4 510 251 (KIRKEMO et al.)<br>* Claims 1,5 *<br>-- | 1,3,5,8 | G 01 N 33/74<br>G 01 N 33/533<br>C 07 J 41/00<br>C 07 J 1/00<br>C 07 J 17/00 |
| X | AT - B - 351 690 (ROUSSEL-UCLAF)<br>* Page 2, lines 1-21 *<br>-- | 1,2,5,6 | |
| X,E | EP - A1 - 0 178 683 (NIHON MEDI-PHYSICS CO., LTD.)<br>* Abstract; claims 1,4,9 *<br>---- | 1,2,4-6 | |

TECHNICAL FIELDS SEARCHED (Int. Cl.4)

G 01 N 33/00
C 07 J

The present search report has been drawn up for all claims

| Place of search | Date of completion of the search | Examiner |
|---|---|---|
| VIENNA | 08-08-1986 | SCHNASS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503 03.82